# EUROPEAN PATENT APPLICATION

(11) **EP 4 821 425 A1**
(43) Date of publication of application: **14.10.2026**
(21) Application number: 25752356.3
(22) Date of filing: 05.02.2025
(51) Int. Cl.: A61B 5/00, A61B 5/11

(54) **ELECTRONIC DEVICE, METHOD, AND NON-TRANSITORY STORAGE MEDIUM FOR IDENTIFYING SLEEP DISORDER**

(30) Priority: 08.02.2024 KR 20240019506
(71) Applicant: Samsung Electronics Co., Ltd., Suwon-si, Gyeonggi-do 16677 (KR)
(72) Inventor: SIM, Heonkyo, Suwon-si Gyeonggi-do 16677 (KR); CHUNG, Gihsung, Suwon-si Gyeonggi-do 16677 (KR); HAN, Jonghee, Suwon-si Gyeonggi-do 16677 (KR)
(74) Representative: Gulde & Partner
(86) International application number: PCT/KR2025/001693
(87) International publication number: WO 2025/170311

(57) **Abstract**

The present document relates to an electronic device, a method, and a non-transitory storage medium for identifying a sleep disorder. According to one embodiment, the electronic device may: acquire first peak signals having a user motion component on the basis of sensing information acquired by an acceleration sensor included in a sensor circuit of the electronic device in relation to periodic limb movements of sleep (PLMS) while a user is sleeping; acquire second peak signals on the basis of first peak signals which satisfy a specified PLMS condition from among the first peak signals; acquire third peak signals by performing interpolation between the second peak signals acquired at specified intervals during a total sleep time; and identify whether there is a PLMS disorder on the basis of third peak signals identified in specified intervals other than a specified interval corresponding to a REM sleep stage from among the third peak signals acquired during the total sleep time. Other embodiments are also possible.

## Description

### [Technical Field]

The disclosure relates to an electronic device, a method, and a non-transitory storage medium for identifying a sleep disorder.

### [Background Art]

Recently, electronic devices have been developed in various forms for the convenience of users, and have been downsized to be conveniently carried by users.

Recently, interest in health has increased, and interest in exercise as a means for maintaining health has also increased. Accordingly, electronic devices have been developed in various forms to measure and utilize various bio signals and movements of the human body, and provide various services for managing a sleep state by identifying a sleep state and a biological state through at least one sensor.

A person sleeps for one-third of his/her lifetime, and if a person fails to get sleep, his/her concentration may decrease and memory may deteriorate, and if a period of insomnia is prolonged, his/her emotions may become unstable and the person may fall into a hallucinatory state. Therefore, since sleep plays a very important role for a healthy life, it is necessary to manage such sleep.

Various sleep disorders may occur during sleep, and among the sleep disorders, there is periodic limb movement of sleep (PLMS). A PLMS disorder had to be identified by visiting a hospital or a sleep examination room. However, despite having a sleep disorder such as a PLMS disorder, there are cases in which it is difficult to enter a sleep examination room and proceed with the examination for various reasons, so that the examination cannot be performed, and thus, conventionally, it has been difficult to easily identify the PLMS disorder.

### [Detailed Description of the Invention]

### [Technical Solution]

According to an embodiment of the disclosure, an electronic device may include a sensor circuit including an acceleration sensor, memory storing instructions, and at least one processor.

According to an embodiment, the instructions may, when executed by the at least one processor, cause the electronic device to obtain first peak signals having a user movement component, based on sensing information obtained in relation to periodic limb movement of sleep (PLMS) by the acceleration sensor while a user is sleeping.

According to an embodiment, the instructions may, when executed by the at least one processor, cause the electronic device to obtain second peak signals based on first peak signals satisfying a specified PLMS condition among the first peak signals.

According to an embodiment, the instructions may, when executed by the at least one processor, cause the electronic device to obtain third peak signals by performing interpolation between second peak signals obtained for each specified section during a total sleep time.

According to an embodiment, the instructions may, when executed by the at least one processor, cause the electronic device to identify whether a PLMS disorder exists based on third peak signals identified in remaining specified sections except for a specified section corresponding to a rapid eye movement (REM) sleep stage among the third peak signals obtained during the total sleep time.

According to an embodiment, an operation method in an electronic device may include obtaining sensing information related to periodic limb movement of sleep (PLMS) by an acceleration sensor included in a sensor circuit of the electronic device while a user is sleeping.

According to an embodiment, the method may include obtaining first peak signals having a user movement component, based on the sensing information.

According to an embodiment, the method may include obtaining second peak signals based on first peak signals satisfying a specified PLMS condition among the first peak signals.

According to an embodiment, the method may include obtaining third peak signals by performing interpolation between second peak signals obtained for each specified section during a total sleep time.

According to an embodiment, the method may include identifying whether a PLMS disorder exists based on third peak signals identified in remaining specified sections except for a specified section corresponding to a rapid eye movement (REM) sleep stage among the third peak signals obtained during the total sleep time.

According to an embodiment, in a non-transitory storage medium storing a program, the program may include executable instructions to cause, when executed by a processor of an electronic device, the electronic device to perform operations of obtaining sensing information related to periodic limb movement of sleep (PLMS) by the acceleration sensor while a user is sleeping, obtaining first peak signals having a user movement component based on the sensing information, obtaining second peak signals based on first peak signals satisfying a specified PLMS condition among the first peak signals, obtaining third peak signals by performing interpolation between second peak signals obtained for each specified section during a total sleep time, and identifying whether a PLMS disorder exists based on third peak signals identified in remaining specified sections except for a specified section corresponding to a rapid eye movement (REM) sleep stage among the third peak signals obtained during the total sleep time.

### [Brief Description of Drawings]

FIG. 1 is a block diagram illustrating an electronic device in a network environment according to various embodiments.
FIG. 2 is a view illustrating a configuration example of an electronic device according to an embodiment.
FIGS. 3A and 3B are views illustrating configuration examples of an electronic device according to an embodiment.
FIGS. 4A and 4B are views illustrating an example for identifying a disorder for periodic limb movement of sleep in an electronic device according to an embodiment.
FIG. 5 is a view illustrating an example for identifying a disorder for periodic limb movement of sleep in an electronic device according to an embodiment.
FIG. 6 is a view illustrating an example for identifying a disorder for periodic limb movement of sleep in an electronic device according to an embodiment.
FIG. 7 is a view illustrating an example for identifying a disorder for periodic limb movement of sleep in an electronic device according to an embodiment.
FIG. 8 is a view illustrating an example of an operation method in an electronic device according to an embodiment.
FIG. 9 is a view illustrating an example of an operation method in an electronic device according to an embodiment.

In relation to the description of the drawings, the same or similar reference numerals may be used for the same or similar components.

### [Mode for Carrying out the Invention]

Hereinafter, embodiments of the disclosure will be described in detail with reference to the drawings so that those skilled in the art may easily implement the disclosure. However, the disclosure may be implemented in various different forms and is not limited to the embodiments described herein. In relation to the description of the drawings, the same or similar reference numerals may be used for the same or similar components. Further, in the drawings and the related descriptions, descriptions of well-known functions and configurations may be omitted for clarity and conciseness. The term "user" used in the embodiments of the disclosure may refer to a person using an electronic device or a device using an electronic device (e.g., an artificial intelligence electronic device).

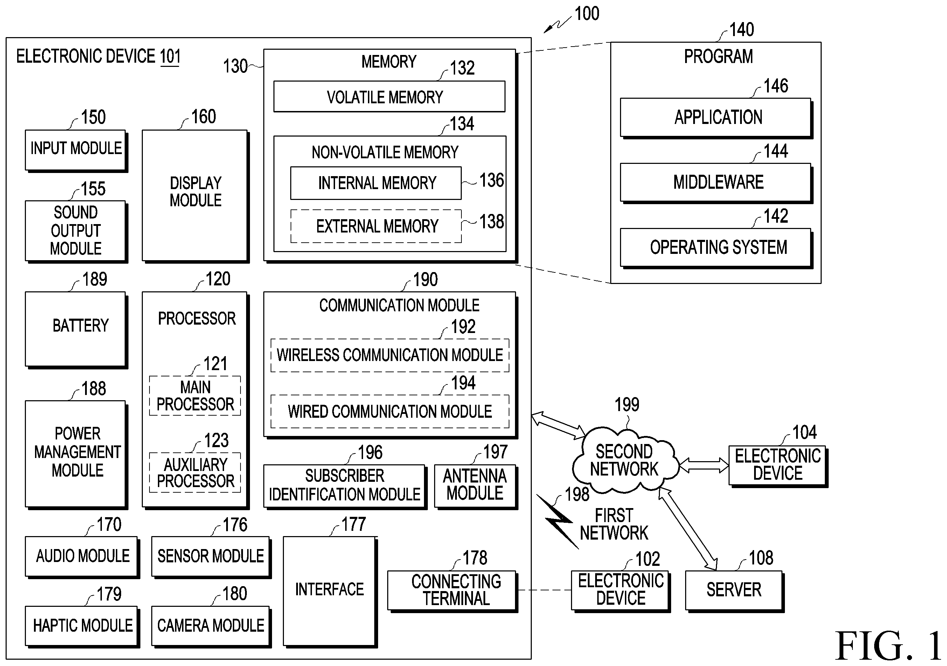

FIG. 1 is a block diagram illustrating an electronic device 101 in a network environment 100 according to various embodiments.

Referring to FIG. 1, in the network environment 100, the electronic device 101 may communicate with an electronic device 102 through a first network 198 (e.g., a short-range wireless communication network), or may communicate with at least one of an electronic device 104 or a server 108 through a second network 199 (e.g., a long-range wireless communication network). According to an embodiment, the electronic device 101 may communicate with the electronic device 104 through the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module 196, or an antenna module 197. In some embodiments, in the electronic device 101, at least one of these components (e.g., the connecting terminal 178) may be omitted, or one or more other components may be added. In some embodiments, some of these components (e.g., the sensor module 176, the camera module 180, or the antenna module 197) may be integrated into a single component (e.g., the display module 160).

The processor 120 may, for example, execute software (e.g., a program 140) to control at least one other component (e.g., a hardware or software component) of the electronic device 101 connected to the processor 120, and may perform various data processing or computations. According to an embodiment, as at least part of the data processing or computation, the processor 120 may store instructions or data received from another component (e.g., the sensor module 176 or the communication module 190) in volatile memory 132, process the instructions or data stored in the volatile memory 132, and store result data in non-volatile memory 134. According to an embodiment, the processor 120 may include a main processor 121 (e.g., a central processing unit or an application processor) or an auxiliary processor 123 (e.g., a graphics processing unit, a neural processing unit (NPU), an image signal processor, a sensor hub processor, or a communication processor) that is operable independently of, or together with, the main processor 121. For example, when the electronic device 101 includes the main processor 121 and the auxiliary processor 123, the auxiliary processor 123 may be configured to use less power than the main processor 121 or to be specialized for a specified function. The auxiliary processor 123 may be implemented separately from, or as a part of, the main processor 121.

The auxiliary processor 123 may, for example, control at least some of functions or states related to at least one component (e.g., the display module 160, the sensor module 176, or the communication module 190) among the components of the electronic device 101 in place of the main processor 121 while the main processor 121 is in an inactive (e.g., sleep) state, or together with the main processor 121 while the main processor 121 is in an active (e.g., application execution) state. According to an embodiment, the auxiliary processor 123 (e.g., the image signal processor or the communication processor) may be implemented as a part of another functionally related component (e.g., the camera module 180 or the communication module 190). According to an embodiment, the auxiliary processor 123 (e.g., the neural processing unit) may include a hardware structure specialized for processing an artificial intelligence model. The artificial intelligence model may be generated through machine learning. Such learning may, for example, be performed by the electronic device 101 itself in which the artificial intelligence model is performed, or may be performed through a separate server (e.g., the server 108). The learning algorithm may include, for example, supervised learning, unsupervised learning, semi-supervised learning, or reinforcement learning, but is not limited to the aforementioned examples. The artificial intelligence model may include a plurality of artificial neural network layers. The artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-networks, or a combination of two or more of the foregoing, but is not limited to the aforementioned examples. The artificial intelligence model may, additionally or alternatively, include a software structure in addition to the hardware structure.

The memory 130 may store various data used by at least one component (e.g., the processor 120 or the sensor module 176) of the electronic device 101. The data may include, for example, software (e.g., the program 140), and input data or output data for instructions related thereto. The memory 130 may include the volatile memory 132 or the non-volatile memory 134.

The program 140 may be stored as software in the memory 130, and may include, for example, an operating system 142, middleware 144, or an application 146.

The input module 150 may receive instructions or data to be used by a component (e.g., the processor 120) of the electronic device 101 from the outside (e.g., a user) of the electronic device 101. The input module 150 may include, for example, a microphone, a mouse, a keyboard, a key (e.g., a button), or a digital pen (e.g., a stylus pen).

The sound output module 155 may output a sound signal to the outside of the electronic device 101. The sound output module 155 may include, for example, a speaker or a receiver. The speaker may be used for general purposes, such as multimedia playback or recording playback. The receiver may be used to receive an incoming call. According to an embodiment, the receiver may be implemented separately from, or as a part of, the speaker.

The display module 160 may visually provide information to the outside (e.g., a user) of the electronic device 101. The display module 160 may include, for example, a display, a hologram device, or a projector, and a control circuit for controlling the corresponding device. According to an embodiment, the display module 160 may include a touch sensor configured to sense a touch, or a pressure sensor configured to measure the intensity of a force generated by the touch.

The audio module 170 may convert sound into an electrical signal, or vice versa, i.e., may convert an electrical signal into sound. According to an embodiment, the audio module 170 may acquire sound through the input module 150, or may output sound through the sound output module 155 or an external electronic device (e.g., the electronic device 102) (e.g., a speaker or headphones) connected directly or wirelessly to the electronic device 101.

The sensor module 176 may sense an operating state (e.g., power or temperature) of the electronic device 101 or an external environmental state (e.g., a user state), and may generate an electrical signal or data value corresponding to the sensed state. According to an embodiment, the sensor module 176 may include, for example, a gesture sensor, a gyro sensor, a barometric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols that may be used for the electronic device 101 to be connected directly or wirelessly to an external electronic device (e.g., the electronic device 102). According to an embodiment, the interface 177 may include, for example, a high definition multimedia interface (HDMI), a universal serial bus (USB) interface, an SD card interface, or an audio interface.

The connecting terminal 178 may include a connector through which the electronic device 101 may be physically connected to an external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., vibration or movement) or an electrical stimulus that a user may perceive through a tactile or kinesthetic sense. According to an embodiment, the haptic module 179 may include, for example, a motor, a piezoelectric element, or an electrical stimulation device.

The camera module 180 may capture still images and videos. According to an embodiment, the camera module 180 may include one or more lenses, image sensors, image signal processors, or flashes.

The power management module 188 may manage power supplied to the electronic device 101. According to an embodiment, the power management module 188 may be implemented, for example, as at least a part of a power management integrated circuit (PMIC).

The battery 189 may supply power to at least one component of the electronic device 101. According to an embodiment, the battery 189 may include, for example, a non-rechargeable primary battery, a rechargeable secondary battery, or a fuel cell.

The communication module 190 may support establishment of a direct (e.g., wired) communication channel or a wireless communication channel between the electronic device 101 and an external electronic device (e.g., the electronic device 102, the electronic device 104, or the server 108), and performance of communication through the established communication channel. The communication module 190 may include one or more communication processors that operate independently of the processor 120 (e.g., an application processor) and support direct (e.g., wired) communication or wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module, or a power line communication module). A corresponding communication module among these communication modules may communicate with an external electronic device 104 through a first network 198 (e.g., a short-range communication network such as Bluetooth, wireless fidelity (Wi-Fi) direct, or infrared data association (IrDA)) or a second network 199 (e.g., a long-range communication network such as a legacy cellular network, a 5G network, a next-generation communication network, the Internet, or a computer network (e.g., LAN or WAN)). These various types of communication modules may be integrated into one component (e.g., a single chip), or may be implemented as a plurality of components (e.g., multiple chips) separate from each other. The wireless communication module 192 may identify or authenticate the electronic device 101 within a communication network, such as the first network 198 or the second network 199, by using subscriber information (e.g., an international mobile subscriber identifier (IMSI)) stored in the subscriber identification module 196.

The wireless communication module 192 may support a 5G network beyond a 4G network, and a next-generation communication technology, for example, a new radio access technology (NR). The NR access technology may support high-speed transmission of high-capacity data (enhanced mobile broadband (eMBB)), terminal power minimization and access of a plurality of terminals (massive machine-type communications (mMTC)), or high reliability and low latency (ultra-reliable and low-latency communications (URLLC)). The wireless communication module 192 may, for example, support a high-frequency band (e.g., an mmWave band) to achieve a high data transmission rate. The wireless communication module 192 may support various technologies for securing performance in the high-frequency band, for example, beamforming, massive multiple-input and multiple-output (MIMO), full-dimensional MIMO (FD-MIMO), an array antenna, analog beamforming, or a large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for realizing 1eMBB, a loss coverage (e.g., 164 dB or less) for realizing mMTC, or a U-plane latency (e.g., 0.5 ms or less for each of downlink (DL) and uplink (UL), or a round trip of 1 ms or less) for realizing URLLC.

The antenna module 197 may transmit a signal or power to the outside (e.g., an external electronic device) or receive it from the outside. According to an embodiment, the antenna module 197 may include an antenna including a radiator made of a conductor or a conductive pattern formed on a substrate (e.g., a PCB). According to an embodiment, the antenna module 197 may include a plurality of antennas (e.g., an array antenna). In such a case, at least one antenna suitable for a communication scheme used in a communication network, such as the first network 198 or the second network 199, may be selected from the plurality of antennas, for example, by the communication module 190. A signal or power may be transmitted or received between the communication module 190 and an external electronic device through the at least one selected antenna. According to some embodiments, in addition to the radiator, another component (e.g., a radio frequency integrated circuit (RFIC)) may additionally be formed as a part of the antenna module 197.

According to various embodiments, the antenna module 197 may form an mmWave antenna module. According to an embodiment, the mmWave antenna module may include a printed circuit board, an RFIC disposed on or adjacent to a first surface (e.g., a lower surface) of the printed circuit board and capable of supporting a specified high-frequency band (e.g., an mmWave band), and a plurality of antennas (e.g., an array antenna) disposed on or adjacent to a second surface (e.g., an upper surface or a side surface) of the printed circuit board and capable of transmitting or receiving signals of the specified high-frequency band.

At least some of the components may be connected to each other through a peripheral device communication scheme (e.g., a bus, general purpose input and output (GPIO), serial peripheral interface (SPI), or mobile industry processor interface (MIPI)) and may exchange signals (e.g., commands or data) with each other.

According to an embodiment, the commands or data may be transmitted or received between the electronic device 101 and the external electronic device 104 through the server 108 connected to the second network 199. Each of the external electronic devices 102 or 104 may be a device of the same type as, or a different type from, the electronic device 101. According to an embodiment, all or some of the operations executed in the electronic device 101 may be executed in one or more external electronic devices among the external electronic devices 102, 104, or 108. For example, when the electronic device 101 needs to perform a certain function or service automatically, or in response to a request from a user or another device, the electronic device 101 may, instead of, or in addition to, executing the function or service by itself, request one or more external electronic devices to perform at least a part of the function or service. The one or more external electronic devices that have received the request may execute at least a part of the requested function or service, or an additional function or service related to the request, and may transfer a result of the execution to the electronic device 101. The electronic device 101 may provide the result, as it is or after additional processing, as at least a part of a response to the request. For this purpose, for example, cloud computing, distributed computing, mobile edge computing (MEC), or client-server computing technology may be used. The electronic device 101 may, for example, provide ultra-low-latency services using distributed computing or mobile edge computing. In another embodiment, the external electronic device 104 may include an Internet of things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology and IoT-related technology.

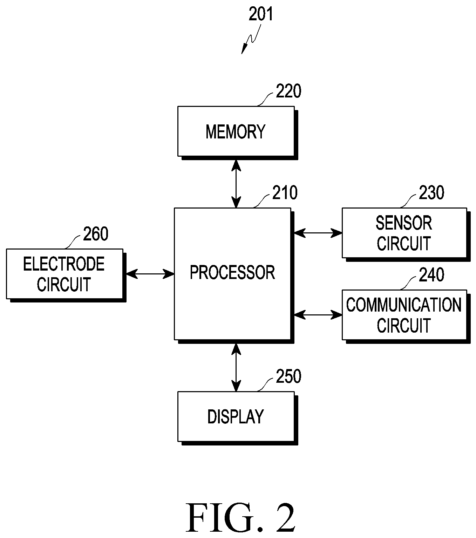

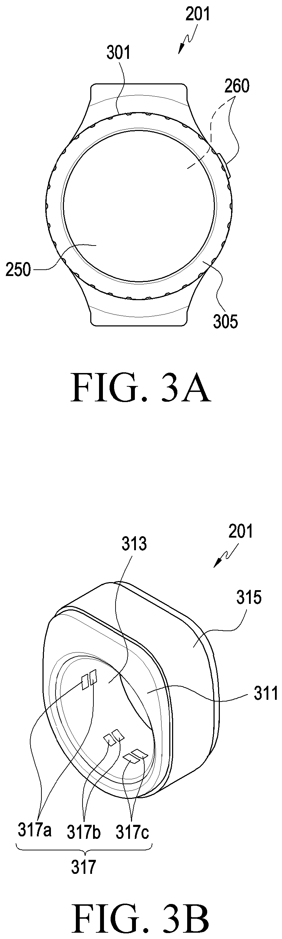

FIG. 2 is a view illustrating a configuration example of an electronic device according to an embodiment, and FIGS. 3A and 3B are views illustrating configuration examples of an electronic device according to an embodiment.

Referring to FIGS. 2, 3A, and 3B, an electronic device 201 (e.g., the electronic device 101 of FIG. 1 or the electronic device 102 or 104) according to an embodiment may include at least one processor 210, memory 220, a sensor circuit 230, a communication circuit 240, a display 250, and an electrode circuit 260. The electronic device 201 is not limited thereto and may be configured to further include various components or to exclude some of the components. The electronic device 201 according to an embodiment may be, e.g., a mobile device carried by a user or a wearable device wearable on a user's body (e.g., a smart watch, a smart band, or a smart ring).

Referring to FIGS. 3A and 3B, according to an embodiment, the electronic device 201 may be, e.g., a wearable device in the form of a wristwatch wearable on a user's wrist or a wearable device (e.g., a ring-type device) wearable on another part of the human body (e.g., a head, a hand, a forearm, a thigh, or another part of the human body capable of measuring bio signals related to sleep). As another example, the electronic device 201 may be another type of user device.

Referring to FIG. 3A, in the electronic device 201 according to an embodiment, the electrode circuit 260 including at least one electrode may be disposed on a first surface (e.g., a rear surface) and a third surface (e.g., a side surface), which are surfaces of a housing 301. In the electronic device 201, the display 250 may be disposed on a second surface (e.g., a front surface), which is another surface of the housing 301. The electronic device 201 may be configured such that at least one sensor is disposed on the first surface so as to be in contact with or proximity to the skin of the human body. The at least one sensor may be included in the sensor circuit 230. For example, the at least one sensor 231 may be a sensor capable of measuring a bio signal.

Referring to FIG. 3B, the electronic device 201 according to an embodiment may be a ring-type wearable device, and may include an outer ring member 311, an inner ring member 313 disposed along an inner circumferential surface of the outer ring member 311, a cover member 315 disposed along an outer circumference of the outer ring member 311, and a circuit unit 317 installed on the outer ring member 311. The circuit unit 317 may include an electrode circuit (e.g., the electrode circuit 260 of FIG. 2) consisting of at least three electrodes 317a, 317b, and 317c. The outer ring member 311 may have an inner diameter that may be fitted on a user's finger. The outer ring member 311 may be manufactured in various sizes to correspond to age, gender, etc., in consideration of various finger thicknesses of multiple users.

The electronic device 201 according to the present embodiments may be configured in various forms in addition to the forms illustrated in FIGS. 3A and 3B.

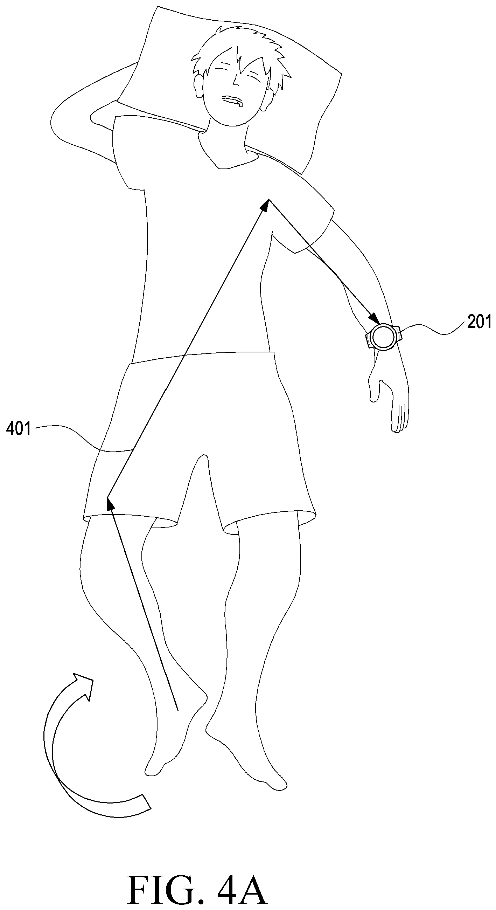

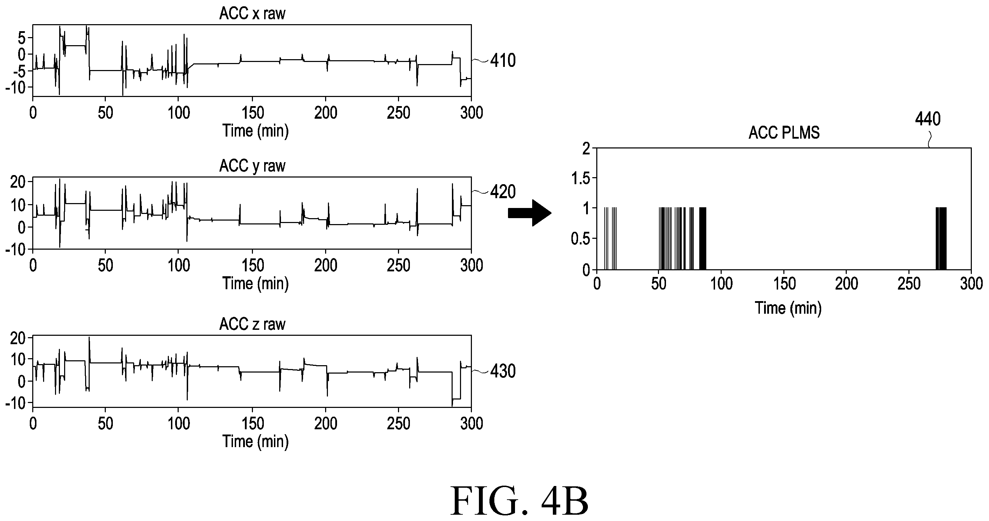

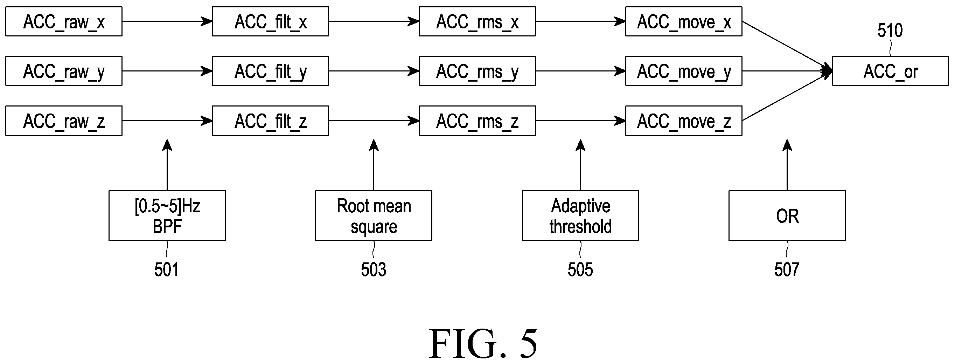

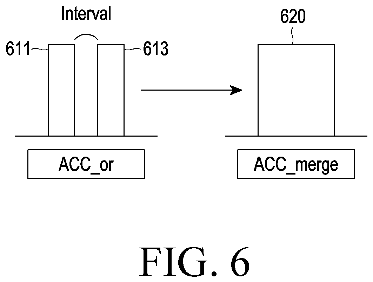

FIGS. 4A and 4B are views illustrating an example for identifying a disorder for periodic limb movement of sleep in an electronic device according to an embodiment. FIGS. 5 to 7 are views illustrating an example for identifying a disorder for periodic limb movement of sleep in an electronic device according to an embodiment.

Referring to FIGS. 2 to 7, according to an embodiment, the processor 210 (e.g., the processor 120 of FIG. 1) of the electronic device 201 may obtain sleep-related information using at least one sensor included in the sensor circuit 230. According to an embodiment, the processor 120 may obtain sleep-related information while a user is sleeping, and store the obtained sleep-related information in the memory 220. According to an embodiment, the processor 210 may obtain sensing information 401 related to periodic limb movement of sleep (PLMS) using an acceleration sensor included in the sensor circuit 230 while a user is sleeping. The sensing information 401 related to periodic limb movement of sleep may be sleep-related information related to a movement (e.g., regular and short muscle activation) of a user's limbs (e.g., arms and/or legs). As another example, the processor 210 may receive information detected by an external electronic device through the communication circuit 240 as sleep-related information. The sleep-related information may include user movement information obtained while a user is sleeping and biometric information detected from the user. Here, the biometric information may include a heart rate (HR) and heart rate variability (HRV). In addition, the biometric information may include electrocardiogram information and/or various bio signals capable of identifying a user's sleep state.

According to an embodiment, the processor 210 may obtain user movement information before a user falls asleep, and automatically detect whether the user has fallen asleep (e.g., sleep onset) based on the obtained movement information. The processor 210 may automatically detect whether a user wakes up (e.g., sleep end) based on user movement information obtained while the user is sleeping. According to an embodiment, the processor 210 may continuously obtain sensing information (e.g., ACC_raw) 401 related to periodic limb movement of sleep during a total sleep time between sleep onset and sleep end. For sleep, one sleep cycle classified into four stages may be repeated during the entire sleep time. The sleep stages during one sleep cycle may be classified into, e.g., a first sleep stage (an awake stage) representing awakening during sleep, a second sleep stage representing light non-rapid-eye-movement (Non-REM) sleep, a third sleep stage representing deep Non-REM sleep, and a fourth sleep stage (a slow wave sleep stage) representing rapid-eye-movement (REM) sleep.

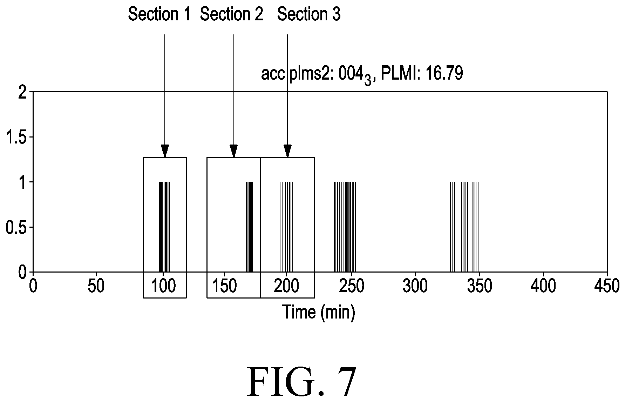

According to an embodiment, the processor 210 may obtain first peak signals (ACC_or) having a user movement component based on the sensing information (ACC_raw) detected using the acceleration sensor. According to an embodiment, when the processor 210 identifies sleep onset, the processor 210 may obtain the sensing information (ACC_raw) detected using the acceleration sensor, and obtain the first peak signals (ACC_or) having a user movement component by shifting, e.g., by 1 second every first time (e.g., 10 seconds), with a specified section (e.g., a section length of approximately 10 minutes). As illustrated in FIG. 4B, the sensing information 401 may include first raw data (ACC_raw_x) 410 of a first axis (e.g., an x-axis), second raw data (ACC_raw_y) 420 of a second axis (e.g., a y-axis), and third raw data (ACC_raw_z) 430 of a third axis (e.g., a z-axis). Here, the specified section is a section length for obtaining the first peak signals (ACC_or) having a user movement component in sections in which PLMS occurs intensively, and the section length may be specified as a predetermined time (e.g., approximately 10 minutes). For example, sections in which PLMS occurs intensively may be represented as section 1 to section 3 of the graph illustrated in FIG. 7, which may be an example configured for convenience of description, and may be represented in another form (or manner).

According to an embodiment, the processor 210 may classify the sensing information (ACC_raw) obtained using the acceleration sensor into first raw data (ACC_raw_x) 410 of a first axis (e.g., an x-axis), second raw data (ACC_raw_y) 420 of a second axis (e.g., a y-axis), and third raw data (ACC_raw_z) 430 of a third axis (e.g., a z-axis). The processor 210 may obtain filtered raw data (ACC_filt_x, ACC_filt_y, ACC_filt_z) of the respective axes by passing the raw data 410, 420, 430 of the respective axes through a band pass filter (e.g., a band pass filter having a cutoff frequency of 0.5 to 5 Hz) 501, and obtain an RMS value (ACC_rms value) by applying a root mean square (RMS) 503 to the obtained filtered raw data of the respective axes (e.g., calculated by shifting the filtered raw data of 1-second length by 0.04 seconds).

According to an embodiment, the processor 210 may identify a value of a movement component (ACC_move_x, ACC_move_y, and ACC_move_z) of each axis based on a predetermined condition for PLMS in raw data (e.g., the root mean square values (ACC_rms_x, ACC_rms_y, and ACC_rms_z) of the respective axes) included in the sensing information. The processor 210 may identify a value of a movement component of each axis by shifting the root mean square values (ACC_rms_x, ACC_rms_y, and ACC_rms_z) of the respective axes by 1 second every first time (e.g., 10 seconds), and obtain (e.g., extract or select) first peak signals (ACC_or) 510 in a specified section based on the identified values of the movement components of the respective axes.

According to an embodiment, the processor 210 may calculate (e.g., obtain) variance for the root mean square values (ACC_rms_x, ACC_rms_y, and ACC_rms_z) of the respective axes obtained during a first time (e.g., 10 seconds), and identify whether the calculated variance is greater than a variance threshold (variance_threshold) 505. The processor 210 may, if the calculated variance is greater than the variance threshold, maintain a previously set resting level value, and if the calculated variance is less than or equal to the variance threshold, set the resting level value as an average of the data detected during the first time (e.g., 10 seconds). For example, the processor 210 may repeatedly perform setting of the resting level value by shifting by 1 second along the time axis. The processor 210 may set a threshold value for a first condition for identifying a movement component using the resting level.

According to an embodiment, the processor 210 may identify (e.g., detect, obtain, or confirm) values (acc_move = 1) of movement components (ACC_move_x, ACC_move_y, and ACC_move_z) of the respective axes that satisfy a predetermined condition for PLMS in raw data (e.g., the root mean square values (ACC_rms_x, ACC_rms_y, and ACC_rms_z) of the respective axes) included in the sensing information. According to an embodiment, the predetermined condition for PLMS may include a first condition that data detected during a first time (e.g., 10 seconds) (e.g., the root mean square values (ACC_rms_x, ACC_rms_y, and ACC_rms_z) of the respective axes) is greater than a threshold value obtained by adding a threshold margin (e.g., a precalculated value of 0.0145) to the resting level value, a second condition that the sleep stage is not a wake stage, and a third condition that the sleep state is in a normal state (e.g., a state that is not a sleep apnea or sleep hypopnea state).

According to an embodiment, when the raw data 410, 420, 430 included in the sensing information satisfies the predetermined condition for PLMS, the processor 210 may identify (e.g., confirm) that the raw data 410, 420, 430 included in the sensing information has a movement component (acc_move = 1). The processor 210 may, when the root mean square values (ACC_rms_x, ACC_rms_y, and ACC_rms_z) of the respective axes satisfy all of the first condition, the second condition, and the third condition, set the values of the movement components (ACC_move_x, ACC_move_y, and ACC_move_z) of the respective axes to a value of 1 indicating that they are movement components related to PLMS.

According to an embodiment, when the predetermined condition for PLMS is not satisfied, the processor 210 may identify that the detected raw data (e.g., the root mean square values (ACC_rms_x, ACC_rms_y, and ACC_rms_z) of the respective axes) included in the sensing information does not have a movement component (acc_move = 0).

According to an embodiment, the processor 210 may, if any one of the first condition, the second condition, or the third condition is not satisfied for the root mean square values (ACC_rms_x, ACC_rms_y, and ACC_rms_z) of the respective axes, set the values of the movement components (ACC_move_x, ACC_move_y, and ACC_move_z) of the respective axes to a value of 0 indicating that they are not movement components related to PLMS.

According to an embodiment, the processor 210 may obtain a value (e.g., 1) of raw data for obtaining a first peak signal (ACC_or) 510 by shifting the values of the movement components (ACC_move_x, ACC_move_y, and ACC_move_z) of the respective axes by 1 second during the first time using an OR gate 507, and obtain (e.g., generate) the first peak signal (ACC_or) 510 having a movement component based on the obtained value of the raw data. The processor 210 may obtain first peak signals having a movement component during the specified section by repeatedly performing the operation for obtaining the first peak signal (ACC_or) 510 as illustrated in FIG. 5 every first time (e.g., 10 seconds) during the specified section.

According to an embodiment, the processor 210 may identify intervals between the first peak signals (ACC_or) obtained during the specified section, and if an interval shorter than a specified interval (e.g., 1.5 seconds) exists among the intervals identified in the specified section, obtain corrected first peak signals (ACC_merge) 620 by combining first peak signals 611, 613 having the corresponding short interval into one signal (e.g., correcting the corresponding short interval to 1).

According to an embodiment, the processor 210 may identify (e.g., confirm, detect, or select) first peak signals corresponding to a specified condition of PLMS among the first peak signals, and obtain (e.g., set, designate, or generate) second peak signals (ACC_plms) 440 in the specified section based on the identified first peak signals.

According to an embodiment, the processor 210 may select first peak signals satisfying a specified condition among the corrected first peak signals (ACC_merge) 620 of the specified section. The processor 210 may maintain (e.g., maintain a value of 1) at least one first peak signal satisfying a condition that a signal generation time (e.g., a time length (x)) is within a first specified range time (0.5 seconds to 10 seconds) (e.g., longer than 0.5 seconds and shorter than 10 seconds) among the corrected first peak signals (ACC_merge) 620, and remove (e.g., set to a value of 0) at least one first peak signal that does not satisfy the condition of being within the first specified range time (0.5 seconds to 10 seconds). The processor 210 may select first peak signals satisfying a condition that an interval of the maintained first peak signals (ACC_cut) in the specified section is less than or equal to a second specified range time (5 seconds to 90 seconds). The processor 210 may obtain selected first peak signals that are continuously repeated a specified number of times (e.g., 4 times) or more in the specified section as second peak signals (ACC_plms) 440 having a value of 1. The processor 210 may remove (e.g., set to a value of 0), in the specified section, at least one of the remaining selected first peak signals that are not continuously repeated the specified number of times (e.g., 4 times) or more.

According to an embodiment, the processor 210 may identify a signal related to apneas and/or hypopneas as a false detection signal based on sleep-related information, and obtain (e.g., count) the number of occurrences of the false detection signal in the specified section. The processor 210 may identify a false detection signal in the corresponding section for each specified section. The processor 210 may, based on the false detection signal being generated more than a specified number of times (e.g., a count value of 5), remove the second peak signals in the specified section (e.g., set ACC_plms_RRLM = 0). A signal related to apneas and/or hypopneas may occur during a respiratory event in which blood oxygen saturation decreases by a certain level (e.g., 3% or more), and may be counted, e.g., when it lasts for 10 seconds.

According to an embodiment, the processor 210 may obtain (e.g., set, designate, or generate) final third peak signals for identifying whether a PLMS disorder exists, after performing interpolation between second peak signals for all sections in units of specified sections during the total sleep time. According to an embodiment, the processor 210 may repeatedly perform an operation of obtaining first peak signals in units of specified sections and obtaining second peak signals based on the first peak signals during the total sleep time, and may perform an operation of identifying second peak signals suitable for identifying whether a PLMS disorder exists among the second peak signals and obtaining final third peak signals for each specified section.

According to an embodiment, the processor 210 may identify intervals between second peak signals remaining after removing the false detection signal in the specified section, and based on some intervals among the identified intervals being greater than a specified interval (1.5 times the median value), perform interpolation between the second peak signals by adding a virtual peak signal to the some intervals. The processor 210 may identify the number (N_interp = round(plms_interval[i]/median(plms_interval))+1) of intervals identified as being greater than the specified interval (1.5 times the median value), and generate virtual peak signals (e.g., virtual PLMS data) by the identified number (N_interp). The processor 210 may maintain the intervals of the second peak signals identified as being less than or equal to the specified interval (1.5 times the median value) as they are.

According to an embodiment, the processor 210 may identify whether intervals (e.g., time intervals) between second peak signals deviate from a third specified range time (12 seconds to 90 seconds), which is a PLMS interval condition, in all sections including the second peak signals during the total sleep time. The processor 210 may, when the interval between the second peak signals is less than or equal to the third specified range time (12 seconds to 90 seconds), maintain the second peak signals, and based on the interval between the second peak signals being greater than the third specified range time (12 seconds to 90 seconds), remove one of the second peak signals existing before and after the interval.

According to an embodiment, the processor 210 may identify a section corresponding to the REM sleep stage among all sections during the total sleep time, and obtain third peak signals (e.g., final peak signals (PLMS signals)) included in the remaining sections except for the identified section. The processor 210 may identify whether a PLMS disorder exists based on the third peak signals included in the remaining sections. The processor 210 may identify whether a PLMS disorder exists using a measure for severity (e.g., a periodic limb movement index (PLMI) defined as the average number of limb movements per sleep hour). The processor 210 may identify the number (e.g., a count value) of third peak signals obtained during the total sleep time, calculate the PLMI by dividing the identified number by the total sleep time, and identify whether a PLMS disorder exists or a PLMS stage based on the calculated PLMI. For example, the processor 210 may, if the calculated PLMI is less than a specified first PLMI index (e.g., 5), identify it as a first PLMS stage as normal. The processor 210 may, if the calculated PLMI is less than a specified second PLMI index (e.g., 5 to 25), identify that there is a mild PLMS disorder as a second PLMS stage. The processor 210 may, if the calculated PLMI is a specified third PLMI index (e.g., 25 to 50), identify that there is a moderate PLMS disorder as a third PLMS stage. The processor 210 may, if the calculated PLMI is greater than or equal to a specified fourth PLMI index (e.g., 50), identify it as a severe PLMS disorder as a fourth PLMS stage.

According to an embodiment, the processor 210 may obtain result information of identifying whether a PLMS disorder exists, and control the display 250 to display the result information. The processor 210 may control the communication circuit 240 to transmit the result information to an external electronic device.

According to an embodiment, the processor 210 may more accurately determine the possession of a PLMS disorder using result information obtained over a certain period (e.g., 3 days, a week, or a month). For example, if the second PLMS stage or higher is identified continuously or a specified number of times during a certain period, the processor 210 may identify that the user has a PLMS disorder, and provide (e.g., display, output, or transmit) guidance information related to the PLMS disorder and/or guidance information related to treatment.

According to an embodiment, the processor 210 may be a hardware module or a software module (e.g., an application program), and may be a hardware component (function) or a software element (program) including at least one of various sensors provided in the electronic device 201, a data measurement module, an input/output interface, a module for managing the state or environment of the electronic device 201, or a communication module.

According to an embodiment, the processor 210 may include, e.g., one or a combination of two or more of hardware, software, or firmware. The processor 210 may be configured to omit at least some of the components, or further include other components for performing image processing operations in addition to the components.

According to an embodiment, the memory 220 (e.g., the memory 130 of FIG. 1) may store an application. For example, the memory 220 may store a sleep-related application (function or program), an exercise application, or a health management application. The memory 220 may store sleep-related information (e.g., user movement information and/or biometric information) obtained through an external electronic device or at least one sensor, sleep state information, sensing information detected by an acceleration sensor in relation to PLMS, peak signals obtained for identifying whether a PLMS disorder exists, and result information of identifying whether a PLMS disorder exists.

According to an embodiment, the memory 220 may store, in addition to a program (e.g., the program 140 of FIG. 1) used for functional operations, various data generated during execution of the program 140. The memory 220 may broadly include a program area 140 and a data area (not illustrated). The program area 140 may store related program information for driving of the electronic device 201, such as an operating system (OS) (e.g., the operating system 142 of FIG. 1) for booting the electronic device 201. The data area (not illustrated) may store data transmitted and/or received and generated data according to various embodiments. Further, the memory 220 may include at least one storage medium of a flash memory, a hard disk, a multimedia card micro type memory (e.g., a secure digital (SD) or extreme digital (XD) memory), a RAM, or a ROM.

According to an embodiment, the sensor circuit 230 (e.g., the sensor module 176 of FIG. 1) may include various sensors for detecting sleep-related information. For example, the sensor circuit 230 may include an acceleration sensor for detecting sensing information related to PLMS. The sensor circuit 230 may include at least one sensor (e.g., a photoplethysmography (PPG) sensor and/or an electrocardiogram (ECG) sensor) for detecting biometric information of a user, at least one sensor (e.g., an acceleration sensor, a proximity sensor, a gyro sensor, and/or a body temperature sensor) 231 for detecting a user's situation, and at least one sensor (e.g., a temperature sensor, a humidity sensor, an illuminance sensor, a camera sensor, a gas sensor, and/or a fine dust sensor) for detecting an external environment of the user. The sensor circuit 230 may further include various sensors for detecting sleep-related information.

According to an embodiment, the communication circuit 240 (e.g., the communication module 190 of FIG. 1) may communicate with an external electronic device (e.g., the electronic device 101 of FIG. 1, the server 108 of FIG. 1, or another user's electronic device). For example, the communication circuit 240 may transmit result information of identifying whether a PLMS disorder exists to an external electronic device. The communication circuit 240 may receive sleep-related information (e.g., movement information, biometric information, and/or sensing information obtained in relation to PLMS) from an external electronic device and/or transmit it to an external electronic device. According to an embodiment, the communication circuit 240 may include a cellular module, a wireless-fidelity (Wi-Fi) module, a Bluetooth module, or a near field communication (NFC) module.

According to an embodiment, the display 250 (e.g., the display module 160 of FIG. 1) may display an execution screen of an application related to a user's sleep. The display 250 may display, under the control of the processor 210, result information of identifying whether a PLMS disorder exists. The display 250 may display, under the control of the processor 210, history information including previously obtained PLMS-related information and/or previously obtained result information. The display 250 may display, under the control of the processor 210, guidance information related to a PLMS disorder.

According to an embodiment, the display 250 may be implemented in the form of a touch screen. The display 250, when implemented together with an input module in the form of a touch screen, may display various pieces of information generated according to a user's touch operation. According to an embodiment, the display 250 may include at least one of a liquid crystal display (LCD), a thin film transistor LCD (TFT-LCD), an organic light emitting diode (OLED), a light emitting diode (LED), an active matrix organic LED (AMOLED), a flexible display, or a 3 dimension display. Further, some of these displays may be configured as a transparent type or a light-transmissive type so that the outside may be seen therethrough. This may be configured in the form of a transparent display including a transparent OLED (TOLED). According to another embodiment, the electronic device may further include another display module (e.g., an extended display or a flexible display) mounted in addition to the display 250.

According to an embodiment, the electrode circuit 260 may include at least one electrode in contact with the human body of a user. For example, the electrode circuit 260 may obtain sleep-related biometric information such as electrocardiogram (ECG) information and bioelectrical impedance analysis (BIA) information from the human body of a user using the electrodes. According to an embodiment, the electronic device 201 may further include an audio circuit (e.g., the audio module 170 of FIG. 1) or a vibration circuit (e.g., the haptic module 179 of FIG. 1). The audio circuit may output sound, and may include, e.g., at least one of an audio codec, a microphone (MIC), a receiver, an earphone output (EAR_L), or a speaker. The audio circuit may output, as an audio signal, result information of identifying whether a user has a PLMS disorder and/or guidance information related to a PLMS disorder obtained based on the result information. For example, the vibration circuit may output, as vibration, result information of identifying whether a user has a PLMS disorder and/or guidance information related to a PLMS disorder obtained based on the result information.

As described above, in an embodiment, the main components of the electronic device have been described through the electronic device 201 of FIG. 2. However, in various embodiments, not all of the components illustrated through FIG. 2 are essential components, and the electronic device 201 may be implemented by more components than the illustrated components, or may be implemented by fewer components. Further, the positions of the main components of the electronic device 201 described above through FIG. 2 may be changeable according to various embodiments.

Referring to FIGS. 1 and 2, an electronic device (e.g., the electronic device 101 of FIG. 1 and/or the electronic device 201 of FIGS. 2, 3A, and 3B) according to an embodiment may implement a sleep-related software module (e.g., the program 140 of FIG. 1) for identifying a PLMS disorder. Memory of the electronic device (e.g., the memory 130 of FIG. 1 and/or the memory 220 of FIG. 2) may store commands (e.g., instructions) for implementing the software module. At least one processor (e.g., the processor 120 of FIG. 1 and/or the processor 210 of FIG. 2) may execute the commands stored in the memory to implement the software module, and may control hardware (e.g., the sensor module 176 of FIG. 1 and/or the sensor circuit 230 of FIG. 2, the communication module 190 of FIG. 1 and/or the communication circuit 240 of FIG. 2, the display module 160 of FIG. 1 and/or the display 250 of FIG. 2) associated with the function of the software module.

The software module of the electronic device according to an embodiment may be configured to include a kernel (or HAL), a framework (e.g., the middleware 144 of FIG. 1), and an application (e.g., the application 146 of FIG. 1). At least a part of the software module may be preloaded on the electronic device 101, or may be downloadable from a server (e.g., the server 108).

According to an embodiment, the kernel may include, e.g., a system resource manager or a device driver, and is not limited thereto and may be configured to further include other modules. The system resource manager may perform control, allocation, or recovery of system resources. The device driver may include, e.g., a display driver, a camera driver, a Bluetooth driver, a shared memory driver, a USB driver, a keypad driver, a WIFI driver, an audio driver, or an inter-process communication (IPC) driver.

According to an embodiment, the framework may provide functions commonly required by an application or various functions to an application through an application programming interface (API) (not illustrated) so that the application may efficiently use limited system resources inside the electronic device. The framework may include modules forming a combination of various functions of the components described above in FIGS. 1 and 2. The framework may provide a module specialized for each type of operating system in order to provide differentiated functions. The framework may dynamically delete some of existing components or add new components.

According to an embodiment, the application may be configured to include a sleep-related application (e.g., a module, a manager, or a program). The application may include an application received from an external electronic device (e.g., the server 108 or the electronic device 102, 104). According to an embodiment, the application may include a preloaded application or a third party application downloadable from a server. The components of the software module and the names of the components according to the illustrated embodiment may vary depending on the type of operating system. According to an embodiment, at least a part of the software module may be implemented as software, firmware, hardware, or a combination of at least two or more thereof. At least a part of the software module may be implemented (e.g., executed) by, e.g., a processor (e.g., an AP). At least a part of the software module may include, e.g., a module, a program, a routine, sets of instructions, or a process for performing at least one function.

As described above, in an embodiment, the main components of the electronic device have been described through the electronic device 101 of FIGS. 1 and 2. However, in various embodiments, not all of the components illustrated through FIGS. 1 and 2 are essential components, and the electronic device 101 may be implemented by more components than the illustrated components, or may be implemented by fewer components. Further, the positions of the main components of the electronic device 101 described above through FIGS. 1 and 2 may be changeable according to various embodiments.

According to an embodiment, an electronic device (e.g., the electronic device 101 of FIG. 1, the electronic device 201 of FIGS. 2, 3A, and 3B) may include a sensor circuit including an acceleration sensor, memory storing instructions (e.g., the memory 130 of FIG. 1, the memory 220 of FIG. 2), and at least one processor (e.g., the processor 120 of FIG. 1, the processor 210 of FIG. 2).

According to an embodiment, the instructions may, when executed by the at least one processor, cause the electronic device to obtain first peak signals having a user movement component, based on sensing information obtained in relation to periodic limb movement of sleep (PLMS) by the acceleration sensor while a user is sleeping.

According to an embodiment, the instructions may, when executed by the at least one processor, cause the electronic device to obtain second peak signals based on first peak signals satisfying a specified PLMS condition among the first peak signals.

According to an embodiment, the instructions may, when executed by the at least one processor, cause the electronic device to obtain third peak signals by performing interpolation between second peak signals obtained for each specified section during a total sleep time.

According to an embodiment, the instructions may, when executed by the at least one processor, cause the electronic device to identify whether a PLMS disorder exists based on third peak signals identified in remaining specified sections except for a specified section corresponding to a REM sleep stage among the third peak signals obtained during the total sleep time.

According to an embodiment, the instructions may, when executed by the at least one processor, cause the electronic device to identify a false detection signal related to apnea or hypopnea for each specified section during the total sleep time, and based on the number of occurrences of the false detection signal is greater than a specified number of times, remove second peak signals identified in correspondence with the false detection signal in a section in which the false detection signal occurred.

According to an embodiment, the instructions may, when executed by the at least one processor, cause the electronic device to identify the number of the third peak signals identified in the remaining specified sections, obtain a periodic limb movement index (PLMI) by dividing the identified number by the total sleep time, and identify whether the PLMS disorder exists and a PLMS stage based on the PLMI.

According to an embodiment, the sensing information may include first raw data (ACC_raw_x) of a first axis (e.g., an x-axis), second raw data (ACC_raw_y) of a second axis (e.g., a y-axis), and third raw data (ACC_raw_z) of a third axis (e.g., a z-axis).

According to an embodiment, the instructions may, when executed by the at least one processor, cause the electronic device to obtain first peak signals every first time in the specified section, based on identifying a user movement component in at least one of the first raw data, the second raw data, or the third raw data.

According to an embodiment, the instructions may, when executed by the at least one processor, cause the electronic device to, based on an interval between the first peak signals being identified as less than a specified time, obtain corrected first peak signals by correcting the interval to a value of the first peak signal.

According to an embodiment, the instructions may, when executed by the at least one processor, cause the electronic device to, based on an interval between the obtained second peak signals being greater than a specified interval, add a virtual peak signal within the interval.

According to an embodiment, the instructions may, when executed by the at least one processor, cause the electronic device to select first peak signals satisfying a condition that a signal generation time is less than or equal to a first specified range time among the first peak signals in the specified section, select first peak signals satisfying a condition that an interval of the first peak signals selected in the specified section is less than or equal to a second specified range time, and obtain selected first peak signals continuously repeated a specified number of times or more in the specified section as the second peak signals having a value of 1.

According to an embodiment, the instructions may, when executed by the at least one processor, cause the electronic device to maintain second peak signals whose interval between the second peak signals is within a third specified range time (12 seconds to 90 seconds) among the obtained second peak signals, and based on the interval being greater than the third specified range time (12 seconds to 90 seconds), remove one of a second peak signal obtained before the interval and/or a second peak signal obtained after the interval.

According to an embodiment, the electronic device may further include a communication circuit (e.g., the communication module 190 of FIG. 1, the communication circuit 240 of FIG. 2).

According to an embodiment, the instructions may, when executed by the at least one processor, cause the electronic device to obtain result information of identifying whether the PLMS disorder exists, control the display to display the result information, and control the communication circuit to transmit the result information to an external electronic device.

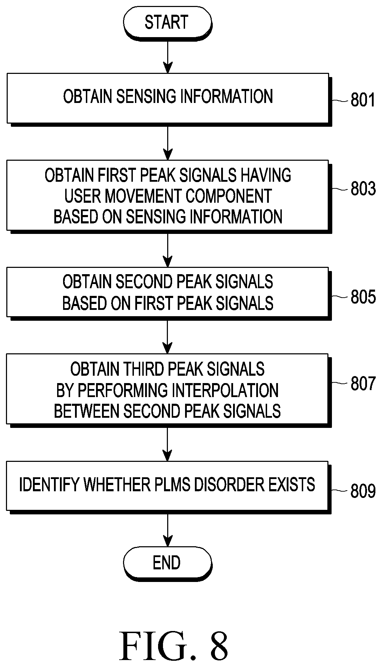

FIG. 8 is a view illustrating an example of an operation method in an electronic device according to an embodiment. In the following embodiments, the operations may be performed sequentially, but are not necessarily performed sequentially. For example, the order of the operations may be changed, and at least two operations may be performed in parallel.

Referring to FIG. 8, in operation 801, an electronic device according to an embodiment (e.g., the electronic device 101 of FIG. 1 and the electronic device 201 of FIGS. 2, 3A, and 3B) may obtain sensing information (ACC_raw) related to periodic limb movement of sleep (PLMS) detected by an acceleration sensor included in a sensor circuit (e.g., the sensor module 176 of FIG. 1 and/or the sensor circuit 230 of FIG. 2) while a user is sleeping. According to an embodiment, the electronic device may receive sensing information detected by an acceleration sensor of an external electronic device worn by the user during sleep, through a communication circuit (e.g., the communication module 190 of FIG. 1 and/or the communication circuit 240 of FIG. 2).

In operation 803, the electronic device according to an embodiment may obtain first peak signals (ACC_or or ACC_merge) having a user movement component in a specified section, based on the obtained sensing information. According to an embodiment, the electronic device may perform an operation of obtaining first peak signals in a specified section (e.g., 10 minutes), and may repeatedly perform the operation in all sections while the user is sleeping.

In operation 805, the electronic device according to an embodiment may obtain (e.g., set) second peak signals (ACC_plms) in the specified section based on first peak signals identified (e.g., selected) corresponding to a specified condition of PLMS among the obtained first peak signals. According to an embodiment, the electronic device may perform an operation of obtaining second peak signals based on the first peak signals (or corrected first peak signals) obtained in the specified section (e.g., 10 minutes), and may repeatedly perform the operation in all sections while the user is sleeping.

In operation 807, the electronic device according to an embodiment may obtain third peak signals by performing interpolation between the second peak signals. The electronic device may obtain third peak signals for each specified section (e.g., 10 minutes) during the total sleep time.

In operation 809, the electronic device according to an embodiment may identify whether a PLMS disorder exists (or a PLMS stage) based on third peak signals (e.g., final peak signals (ACC_plms_REM)) included in the remaining sections except for a section corresponding to a REM sleep stage among all specified sections within the total sleep time. According to an embodiment, the electronic device may perform an operation of identifying whether a PLMS disorder exists (or a PLMS stage) after the end of sleep.

By the operation method of FIG. 8 described above, the electronic device according to an embodiment may obtain result information of identifying whether a PLMS disorder exists, and display the obtained result information on the display and/or transmit it to an external electronic device through a communication circuit. The electronic device may more accurately determine the possession of a PLMS disorder using result information obtained over a certain period (e.g., 3 days, a week, or a month). For example, if the second PLMS stage or higher is identified continuously or a specified number of times during a certain period, the electronic device may identify that the user has a PLMS disorder, and provide guidance information related to the PLMS disorder and/or guidance information related to treatment.

The electronic device according to an embodiment may execute a sleep-related application for PLMS, and may be a wearable device wearable on a user's body, but is not limited thereto, and the electronic device may execute a sleep-related application for PLMS, and may be a user device (e.g., a mobile phone) capable of communicating with a wearable device worn by the user. Such a user device may receive sensing information detected by an acceleration sensor from a wearable device worn by the user during sleep, and identify whether a PLMS disorder exists by performing operations 803 to 807 using the received sensing information.

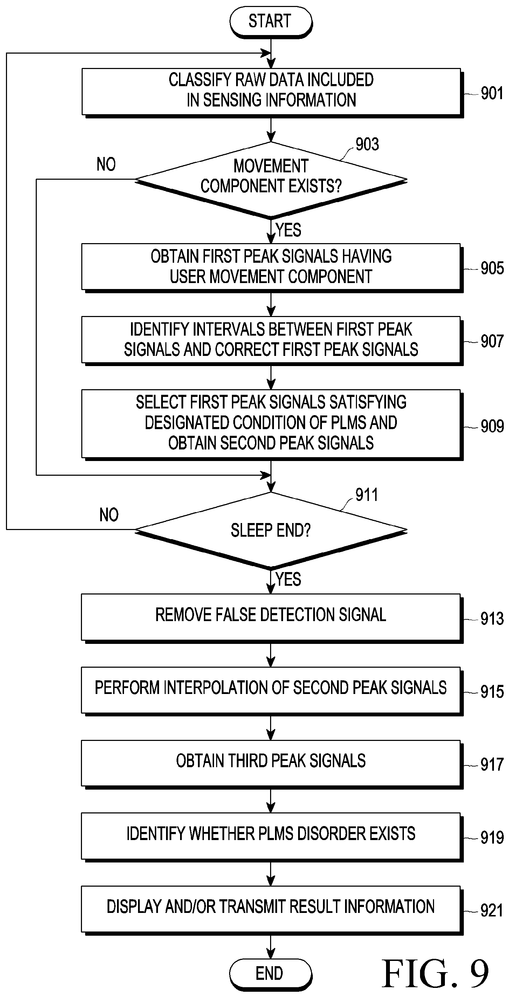

FIG. 9 is a view illustrating an example of an operation method in an electronic device according to an embodiment. In the following embodiments, the operations may be performed sequentially, but are not necessarily performed sequentially. For example, the order of the operations may be changed, and at least two operations may be performed in parallel.

Referring to FIG. 9, an electronic device according to an embodiment (e.g., the electronic device 101 of FIG. 1 and the electronic device 201 of FIGS. 2, 3A, and 3B) may obtain sensing information (ACC_raw) related to periodic limb movement of sleep (PLMS) detected by an acceleration sensor included in a sensor circuit (e.g., the sensor module 176 of FIG. 1 and/or the sensor circuit 230 of FIG. 2) while a user is sleeping, as in operation 801 of FIG. 8. According to an embodiment, the electronic device may obtain the sensing information from an acceleration sensor of an external electronic device (e.g., a wearable electronic device) worn by the user. The sensing information (e.g., the sensing information 401 of FIG. 4) may include first raw data (ACC_raw_x) of a first axis (e.g., an x-axis), second raw data (ACC_raw_y) of a second axis (e.g., a y-axis), and third raw data (ACC_raw_z) of a third axis (e.g., a z-axis), as illustrated in FIG. 4B.

In operation 901, according to an embodiment, the electronic device may classify the sensing information (ACC_raw) into first raw data (ACC_raw_x) of a first axis (e.g., an x-axis), second raw data (ACC_raw_y) of a second axis (e.g., a y-axis), and third raw data (ACC_raw_z) of a third axis (e.g., a z-axis). According to an embodiment, the electronic device may obtain filtered raw data (ACC_filt_x, ACC_filt_y, ACC_filt_z) of the respective axes by passing the raw data (ACC_raw_x, ACC_raw_y, ACC_raw_z) of the respective axes through a band pass filter (e.g., a band pass filter having a cutoff frequency of 0.5 to 5 Hz) 501, and obtain root mean square values (ACC_rms values) for the obtained filtered raw data of the respective axes (e.g., calculated by shifting the filtered raw data of 1-second length by 0.04 seconds). According to an embodiment, the electronic device may calculate (e.g., obtain) variance for the root mean square values (ACC_rms_x, ACC_rms_y, and ACC_rms_z) of the respective axes obtained during a first time (e.g., 10 seconds), and identify whether the calculated variance is greater than a variance threshold (variance_threshold). The electronic device may, if the calculated variance is greater than the variance threshold, maintain a previously set resting level value, and if the calculated variance is less than or equal to the variance threshold, set the resting level value as an average of the data detected during the first time (e.g., 10 seconds). For example, the processor 210 may repeatedly perform setting of the resting level value by shifting by 1 second along the time axis. The electronic device may set a threshold value (e.g., a value obtained by adding a threshold margin to the resting level value) for a predetermined condition (e.g., a first condition) for identifying a movement component using the resting level. Here, the predetermined condition for PLMS may include a first condition that data detected during a first time (e.g., 10 seconds) (e.g., the root mean square values (ACC_rms_x, ACC_rms_y, and ACC_rms_z) of the respective axes) is greater than a threshold value obtained by adding a threshold margin (e.g., a precalculated value of 0.0145) to the resting level value, a second condition that the sleep stage is not a wake stage, and a third condition that the sleep state is in a normal state (e.g., a state that is not a sleep apnea or sleep hypopnea state). According to an embodiment, the electronic device may identify whether the raw data of the respective axes (e.g., the root mean square values (ACC_rms_x, ACC_rms_y, and ACC_rms_z) of the respective axes) satisfies a predetermined condition for PLMS, respectively, and if each raw data does not satisfy the predetermined condition, identify that there is no movement component (acc_move = 0), and if the predetermined condition is satisfied, identify that each raw data has a movement component (acc_move = 1).

In operation 903, the electronic device may identify whether a user movement component exists in raw data of at least one axis among the raw data of the respective axes classified from the sensing information detected by shifting, e.g., by 1 second every first time (e.g., 10 seconds) in a specified section (e.g., 10 minutes). In operation 903, the electronic device according to an embodiment may identify whether a user movement component exists in raw data of at least one axis among the raw data of the respective axes using an OR gate. As a result of the identification, if a movement component exists in at least one of the raw data of the respective axes, the electronic device may perform operation 905. As a result of the identification, if a movement component does not exist in at least one of the raw data of the respective axes (e.g., when there is no movement component in all raw data of the respective axes), the electronic device may not generate first peak signals (ACC_or) having a user movement component, and may perform operation 911.

In operation 905 (operation 903-Yes), the electronic device may obtain (e.g., generate or set) first peak signals (ACC_or) having a user movement component, based on identifying a user movement component in raw data of at least one axis among the raw data of the respective axes.

In operation 907, the electronic device may correct the first peak signals by identifying intervals between the first peak signals (ACC_or) obtained during the specified section. The electronic device may, if an interval shorter than a specified interval (e.g., 1.5 seconds) exists among the intervals between the first peak signals identified in the specified section, obtain corrected first peak signals (acc_merge) by combining the first peak signals having the corresponding short interval into one signal (e.g., correcting the corresponding short interval to 1).

In operation 909, the electronic device may identify (e.g., identify, detect, or select) first peak signals satisfying a specified condition of PLMS among the first peak signals, and obtain (e.g., set, designate, or generate) second peak signals (ACC_plms) in the specified section based on the identified first peak signals. According to an embodiment, the electronic device may maintain (e.g., maintain a value of 1) at least one first peak signal satisfying a condition that a signal generation time (e.g., a time length (x)) is within a first specified range time (0.5 seconds to 10 seconds) (e.g., longer than 0.5 seconds and shorter than 10 seconds) among the corrected first peak signals (acc_merge), and remove (e.g., set to a value of 0) at least one first peak signal that does not satisfy the condition of being within the first specified range time (0.5 seconds to 10 seconds). The electronic device may select first peak signals satisfying a condition that an interval of the maintained first peak signals (ACC_cut) in the specified section is less than or equal to a second specified range time (5 seconds to 90 seconds). The processor 210 may obtain selected first peak signals continuously repeated a specified number of times (e.g., 4 times) or more in the specified section as second peak signals (ACC_plms) having a value of 1. The processor 210 may remove (e.g., set to a value of 0), in the specified section, at least one of the remaining selected first peak signals that are not continuously repeated the specified number of times (e.g., 4 times) or more.

Operations 901 to 909 described above are operations repeatedly performed every first time (e.g., 10 seconds) during a first section (e.g., approximately 10 minutes), and the first peak signals (ACC_or) having a user movement component and the second peak signals (ACC_plms) may be signals obtained during the first section (e.g., approximately 10 minutes).

In operation 911 (operation 903-No), the electronic device may identify whether sleep has ended. As a result of the identification, if sleep has not ended, the electronic device may perform operation 901 again. If the end of sleep is identified, the electronic device may perform operation 913.

In operation 913 (operation 911-Yes), the electronic device may identify a signal related to apneas and/or hypopneas as a false detection signal based on sleep-related information, and obtain (e.g., count) the number of occurrences of the false detection signal. The electronic device may identify a false detection signal in the corresponding section for each specified section in the total sleep time, and remove the identified false detection signal in the corresponding section. The electronic device may, based on the false detection signal being generated more than a specified number of times (e.g., a count value of 5), identify that the second peak signals obtained in the corresponding section correspond to the falsely detected signal, and remove the second peak signals corresponding to the false detection signal (e.g., set ACC_plms_RRLM = 0). A signal related to apneas and/or hypopneas may occur during a respiratory event in which blood oxygen saturation decreases by a certain level (e.g., 3% or more), and may be counted, e.g., when it lasts for 10 seconds. In the operation method of FIG. 9, although operation 913 has been described as being performed after the end of sleep, it is not limited thereto, and operation 913 may be performed for each specified section while a user is sleeping before the sleep end operation (operation 911).

In operation 915, the electronic device may perform interpolation between the second peak signals for all sections in units of specified sections in the total sleep time. According to an embodiment, the electronic device may identify intervals between second peak signals remaining after removing the false detection signal in the specified section, and based on some intervals among the identified intervals being greater than a specified interval (1.5 times the median value), perform interpolation between the second peak signals by adding a virtual peak signal to the some intervals. The electronic device may identify the number (N_interp = round(plms_interval[i]/median(plms_interval))+1) of intervals identified as being greater than the specified interval (1.5 times the median value), and generate virtual peak signals (e.g., virtual PLMS data) by the identified number (N_interp). The processor 210 may maintain the intervals of the second peak signals identified as being less than or equal to the specified interval (1.5 times the median value) as they are. According to an embodiment, the electronic device may identify whether intervals (e.g., time intervals) between second peak signals deviate from a third specified range time (12 seconds to 90 seconds), which is a PLMS interval condition, in all sections including the second peak signals during the total sleep time. The processor 210 may, when the interval between the second peak signals is less than or equal to the third specified range time (12 seconds to 90 seconds), maintain the second peak signals, and based on the interval between the second peak signals being greater than the third specified range time (12 seconds to 90 seconds), remove one of the second peak signals existing before and after the interval.

In operation 917, the electronic device may obtain (e.g., set, designate, or generate) final third peak signals for identifying whether a PLMS disorder exists, based on the interpolated second peak signals.

In operation 919, the electronic device may identify a section corresponding to the REM sleep stage among all sections during the total sleep time, and identify whether a PLMS disorder exists based on third peak signals (e.g., final peak signals (PLMS signals)) included in the remaining sections except for the identified section. According to an embodiment, the electronic device may identify whether a PLMS disorder exists using a measure for severity (e.g., a periodic limb movement index (PLMI) defined as the average number of limb movements per sleep hour). The electronic device may identify the number (e.g., a count value) of third peak signals obtained during the total sleep time, calculate the PLMI by dividing the identified number by the total sleep time, and identify whether a PLMS disorder exists or a PLMS stage based on the calculated PLMI. For example, the electronic device may, if the calculated PLMI is less than a specified first PLMI index (e.g., 5), identify it as a first PLMS stage as normal. The electronic device may, if the calculated PLMI is less than a specified second PLMI index (e.g., 5 to 25), identify that there is a mild PLMS disorder as a second PLMS stage. The electronic device may, if the calculated PLMI is a specified third PLMI index (e.g., 25 to 50), identify that there is a moderate PLMS disorder as a third PLMS stage. The electronic device may, if the calculated PLMI is greater than or equal to a specified fourth PLMI index (e.g., 50), identify it as a severe PLMS disorder as a fourth PLMS stage.

In operation 921, according to an embodiment, the electronic device may obtain result information of identifying whether a PLMS disorder exists, and control the display 250 to display the result information on the display. The electronic device may control the communication circuit 240 to transmit the result information to an external electronic device. Thereafter, the electronic device may end the operation.

According to an embodiment, the electronic device may more accurately determine the possession of a PLMS disorder using result information obtained over a certain period (e.g., 3 days, a week, or a month). For example, if the second PLMS stage or higher is identified continuously or a specified number of times during a certain period, the electronic device may identify that the user has a PLMS disorder, and provide (e.g., display, output, or transmit) guidance information related to the PLMS disorder and/or guidance information related to treatment.

According to an embodiment, an operation method in an electronic device (e.g., the electronic device 101 of FIG. 1 and the electronic device 201 of FIGS. 2, 3A, and 3B) may include obtaining sensing information related to periodic limb movement of sleep (PLMS) by an acceleration sensor included in a sensor circuit (e.g., the sensor module 176 of FIG. 1, the sensor circuit 230 of FIG. 2) of the electronic device while a user is sleeping.

According to an embodiment, the method may include obtaining first peak signals having a user movement component, based on the sensing information.

According to an embodiment, the method may include obtaining second peak signals based on first peak signals satisfying a specified PLMS condition among the first peak signals.

According to an embodiment, the method may include obtaining third peak signals by performing interpolation between second peak signals obtained for each specified section during a total sleep time.

According to an embodiment, the method may include identifying whether a PLMS disorder exists based on third peak signals identified in remaining specified sections except for a specified section corresponding to a REM sleep stage among the third peak signals obtained during the total sleep time.

According to an embodiment, the method may further include identifying a false detection signal related to apnea or hypopnea for each specified section during the total sleep time, and based on the number of occurrences of the false detection signal is greater than a specified number of times, removing second peak signals identified in correspondence with the false detection signal in a section in which the false detection signal occurred.

According to an embodiment, the sensing information may include first raw data (ACC_raw_x) of a first axis (e.g., an x-axis), second raw data (ACC_raw_y) of a second axis (e.g., a y-axis), and third raw data (ACC_raw_z) of a third axis (e.g., a z-axis).

According to an embodiment, the method may include obtaining first peak signals every first time in the specified section, based on identifying a user movement component in at least one of the first raw data, the second raw data, or the third raw data.

According to an embodiment, the method may further include, based on an interval between the first peak signals being identified as less than a specified time, obtaining corrected first peak signals by correcting the interval to a value of the first peak signal.

According to an embodiment, the method may further include, based on an interval between the obtained second peak signals being greater than a specified interval, adding a virtual peak signal within the interval.

According to an embodiment, the obtaining of the second peak signals may include selecting first peak signals satisfying a condition that a signal generation time is less than or equal to a first specified range time among the first peak signals in the specified section, selecting first peak signals satisfying a condition that an interval of the first peak signals selected in the specified section is less than or equal to a second specified range time, and obtaining selected first peak signals continuously repeated a specified number of times or more in the specified section as the second peak signals having a value of 1.

According to an embodiment, the obtaining of the third peak signals by performing interpolation between the second peak signals may include maintaining second peak signals whose interval between the second peak signals is within a third specified range time (12 seconds to 90 seconds) among the obtained second peak signals, and based on the interval being greater than the third specified range time (12 seconds to 90 seconds), removing one of a second peak signal obtained before the interval and/or a second peak signal obtained after the interval.

According to an embodiment, the method may further include obtaining result information of identifying whether the PLMS disorder exists, and displaying the result information on the display.

According to an embodiment, the method may further include transmitting the result information to an external electronic device through a communication circuit of the electronic device.

According to an embodiment, in a non-transitory storage medium storing a program, the program may include executable instructions to cause, when executed by a processor of an electronic device, the electronic device to perform operations of obtaining sensing information related to periodic limb movement of sleep (PLMS) by the acceleration sensor while a user is sleeping, obtaining first peak signals having a user movement component based on the sensing information, obtaining second peak signals based on first peak signals satisfying a specified PLMS condition among the first peak signals, obtaining third peak signals by performing interpolation between second peak signals obtained for each specified section during a total sleep time, and identifying whether a PLMS disorder exists based on third peak signals identified in remaining specified sections except for a specified section corresponding to a REM sleep stage among the third peak signals obtained during the total sleep time.

According to an embodiment, the disclosure may detect a PLMS disorder early by easily measuring PLMS, which could be measured with an EMG sensor in a hospital, at home through an electronic device (e.g., a wearable device) by obtaining and correcting peak signals having a movement component and satisfying the PLMS condition using sensing information detected by an acceleration sensor and performing signal processing appropriate for PLMS. In addition, various effects directly or indirectly identified through the present document may be provided. The effects obtainable from the disclosure are not limited to the effects mentioned above, and other effects not mentioned may be clearly understood by those skilled in the art to which the disclosure pertains from the description below.

The embodiments disclosed in the present document are presented for description and understanding of the disclosed technical content, and do not limit the scope of the technology described in the present document. Therefore, the scope of the present document should be construed to include all changes or various other embodiments based on the technical idea of the present document.

The electronic device according to various embodiments disclosed in the present document may be one of various types of devices. The electronic device may include, e.g., a portable communication device (e.g., a smart phone), a computer device, a portable multimedia device, a portable medical device, a camera, a wearable device, or a home appliance device. The electronic device according to embodiments of the present document is not limited to the aforementioned devices.

The various embodiments of the present document and the terms used herein are not intended to limit the technical features described in the present document to specific embodiments, and should be understood to include various changes, equivalents, or replacements of the corresponding embodiments. In connection with the description of the drawings, similar reference numerals may be used for similar or related components. The singular form of a noun corresponding to an item may include one or more of the items, unless the relevant context clearly indicates otherwise. In the present document, each of the phrases such as "A or B," "at least one of A and B," "at least one of A or B," "A, B or C," "at least one of A, B and C," and "at least one of A, B, or C" may include any one of the items listed together in the corresponding phrase, or all possible combinations thereof. Terms such as "first" or "second" may be used merely to distinguish a corresponding component from another corresponding component, and do not limit the corresponding components in another aspect (e.g., importance or order). When a (e.g., first) component is referred to as being "coupled" or "connected" to another (e.g., second) component, with or without the term "functionally" or "communicatively," it means that the certain component may be connected to the other component directly (e.g., by wire), wirelessly, or through a third component.

The term "module" used in various embodiments of the present document may include a unit implemented in hardware, software, or firmware, and may be used interchangeably with terms such as logic, logic block, component, or circuit. A module may be an integrally configured component or a minimum unit or a part thereof of the component performing one or more functions. For example, according to an embodiment, the module may be implemented in the form of an application-specific integrated circuit (ASIC).

The various embodiments of the present document may be implemented as software (e.g., a program 140) including one or more instructions stored in a storage medium (e.g., the internal memory 136 or the external memory 138) readable by a machine (e.g., the electronic device 101). For example, a processor (e.g., the processor 120) of the machine (e.g., the electronic device 101) may invoke at least one of the one or more instructions stored from the storage medium and execute it. This makes it possible for the machine to be operated to perform at least one function according to the at least one invoked instruction. The one or more instructions may include code generated by a compiler or code that may be executed by an interpreter. The machine-readable storage medium may be provided in the form of a non-transitory storage medium. Here, "non-transitory" merely means that the storage medium is a tangible device and does not include a signal (e.g., an electromagnetic wave), and this term does not distinguish between a case where data is stored semi-permanently in the storage medium and a case where data is temporarily stored.

According to an embodiment, the method according to an embodiment disclosed in the present document may be provided by being included in a computer program product. The computer program product may be traded as a commodity between a seller and a buyer. The computer program product may be distributed in the form of a machine-readable storage medium (e.g., a compact disc read only memory (CD-ROM)), or distributed (e.g., downloaded or uploaded) online through an application store (e.g., Play Store^{™}) or directly between two user devices (e.g., smart phones). In the case of online distribution, at least a part of the computer program product may be at least temporarily stored or temporarily generated in a machine-readable storage medium such as memory of a manufacturer's server, a server of an application store, or a relay server.

According to various embodiments, each component (e.g., a module or a program) of the aforementioned components may include a single entity or a plurality of entities, and some of the plurality of entities may be separately disposed in another component. According to various embodiments, one or more of the aforementioned components or operations may be omitted, or one or more other components or operations may be added. Alternatively or additionally, a plurality of components (e.g., modules or programs) may be integrated into one component. In this case, the integrated component may perform one or more functions of each component of the plurality of components in the same or similar manner as performed by a corresponding component among the plurality of components prior to the integration. According to various embodiments, operations performed by a module, a program, or another component may be executed sequentially, in parallel, repeatedly, or heuristically, or one or more of the operations may be executed in a different order or omitted, or one or more other operations may be added.

## Claims

1. An electronic device (101, 201) comprising:
a sensor circuit (176, 230) including an acceleration sensor;
memory (130, 220) storing instructions; and
at least one processor (120, 210), wherein the instructions, when executed by the at least one processor, cause the electronic device to:
obtain first peak signals having a user movement component based on sensing information obtained in relation to periodic limb movement of sleep (PLMS) by the acceleration sensor while a user is sleeping;
obtain second peak signals based on first peak signals satisfying a specified PLMS condition among the first peak signals;
obtain third peak signals by performing interpolation between second peak signals obtained for each specified section during a total sleep time; and
identify whether a PLMS disorder exists based on third peak signals identified in remaining specified sections except for a specified section corresponding to a REM sleep stage among the third peak signals obtained during the total sleep time.

2. The electronic device of claim 1, wherein the instructions, when executed by the at least one processor, cause the electronic device to:
identify a false detection signal related to apnea or hypopnea for each specified section during the total sleep time, and based on the number of occurrences of the false detection signal is greater than a specified number of times, remove second peak signals identified in correspondence with the false detection signal in a section in which the false detection signal occurred.

3. The electronic device of claim 1 or 2, wherein the instructions, when executed by the at least one processor, cause the electronic device to:
identify the number of the third peak signals identified in the remaining specified sections;
obtain a periodic limb movement index (PLMI) by dividing the identified number by the total sleep time; and
identify whether the PLMS disorder exists and a PLMS stage based on the PLMI,
wherein the sensing information includes first raw data (ACC_raw_x) of a first axis, second raw data (ACC_raw_y) of a second axis, and third raw data (ACC_raw_z) of a third axis.

4. The electronic device of any one of claims 1 to 3, wherein the instructions, when executed by the at least one processor, cause the electronic device to:
obtain first peak signals every first time in the specified section, based on identifying a user movement component in at least one of the first raw data, the second raw data, or the third raw data.

5. The electronic device of any one of claims 1 to 4, wherein the instructions, when executed by the at least one processor, cause the electronic device to:
based on an interval between the first peak signals being identified as less than a specified time, obtain corrected first peak signals by correcting the interval to a value of the first peak signal; and
based on an interval between the obtained second peak signals being greater than a specified interval, add a virtual peak signal within the interval.

6. The electronic device of any one of claims 1 to 5, wherein the instructions, when executed by the at least one processor, cause the electronic device to:
select first peak signals satisfying a condition that a signal generation time is less than or equal to a first specified range time among the first peak signals in the specified section;
select first peak signals satisfying a condition that an interval of the first peak signals selected in the specified section is less than or equal to a second specified range time; and
obtain selected first peak signals continuously repeated a specified number of times or more in the specified section as the second peak signals having a value of 1.

7. The electronic device of any one of claims 1 to 6, wherein the instructions, when executed by the at least one processor, cause the electronic device to:
maintain second peak signals whose interval between the second peak signals is within a third specified range time among the obtained second peak signals; and
based on the interval being greater than the third specified range time, remove one of a second peak signal obtained before the interval and/or a second peak signal obtained after the interval.

8. The electronic device of any one of claims 1 to 9, further comprising:
a display (160, 250); and
a communication circuit (190, 240), wherein the instructions, when executed by the at least one processor, cause the electronic device to:
obtain result information of identifying whether the PLMS disorder exists;
control the display to display the result information; and
control the communication circuit to transmit the result information to an external electronic device.

9. An operation method in an electronic device (101, 201), comprising:
obtaining sensing information related to periodic limb movement of sleep (PLMS) by an acceleration sensor included in a sensor circuit (176, 230) of the electronic device while a user is sleeping;
obtaining first peak signals having a user movement component, based on the sensing information;
obtaining second peak signals based on first peak signals satisfying a specified PLMS condition among the first peak signals;
obtaining third peak signals by performing interpolation between second peak signals obtained for each specified section during a total sleep time; and
identifying whether a PLMS disorder exists based on third peak signals identified in remaining specified sections except for a specified section corresponding to a REM sleep stage among the third peak signals obtained during the total sleep time.

10. The method of claim 9, further comprising:
identifying a false detection signal related to apnea or hypopnea for each specified section during the total sleep time; and
based on the number of occurrences of the false detection signal is greater than a specified number of times, removing second peak signals identified in correspondence with the false detection signal in a section in which the false detection signal occurred, wherein the sensing information includes first raw data (ACC_raw_x) of a first axis, second raw data (ACC_raw_y) of a second axis, and third raw data (ACC_raw_z) of a third axis.

11. The method of claim 9 or 10, wherein the obtaining of the first peak signals comprises:
obtaining first peak signals every first time in the specified section, based on identifying a user movement component in at least one of the first raw data, the second raw data, or the third raw data.

12. The method of any one of claims 9 to 11, further comprising:
based on an interval between the first peak signals being identified as less than a specified time, obtaining corrected first peak signals by correcting the interval to a value of the first peak signal; and
based on an interval between the obtained second peak signals being greater than a specified interval, adding a virtual peak signal within the interval.

13. The method of any one of claims 9 to 12, wherein the obtaining of the second peak signals comprises:
selecting first peak signals satisfying a condition that a signal generation time is less than or equal to a first specified range time among the first peak signals in the specified section;
selecting first peak signals satisfying a condition that an interval of the first peak signals selected in the specified section is less than or equal to a second specified range time; and
obtaining selected first peak signals continuously repeated a specified number of times or more in the specified section as the second peak signals having a value of 1.

14. The method of any one of claims 9 to 13, wherein the obtaining of the third peak signals by performing the interpolation between the second peak signals comprises:
maintaining second peak signals whose interval between the second peak signals is within a third specified range time among the obtained second peak signals; and
based on the interval being greater than the third specified range time, removing one of a second peak signal obtained before the interval and/or a second peak signal obtained after the interval,
the method further comprising:
obtaining result information of identifying whether the PLMS disorder exists;
displaying the result information on a display (160, 250) of the electronic device; and
transmitting the result information to an external electronic device through a communication circuit (190, 240) of the electronic device.

15. A non-transitory storage medium storing a program, the program comprising executable instructions to cause, when executed by at least one processor (120, 210) of an electronic device, the electronic device (101, 201) to perform:
obtaining sensing information related to periodic limb movement of sleep (PLMS) by the acceleration sensor while a user is sleeping;
obtaining first peak signals having a user movement component, based on the sensing information;
obtaining second peak signals based on first peak signals satisfying a specified PLMS condition among the first peak signals;
obtaining third peak signals by performing interpolation between second peak signals obtained for each specified section during a total sleep time; and
identifying whether a PLMS disorder exists based on third peak signals identified in remaining specified sections except for a specified section corresponding to a REM sleep stage among the third peak signals obtained during the total sleep time.
